# EUROPEAN PATENT APPLICATION

(11) **EP 4 020 493 A2**
(43) Date of publication of application: **29.06.2022**
(21) Application number: 20878640.0
(22) Date of filing: 22.12.2020
(51) Int. Cl.: G16H 50/20, G16H 30/20

(54) **SEVERITY ASSESSMENT DEVICE AND MODEL GENERATION DEVICE**

(30) Priority: 25.10.2019 JP 2019194633
(71) Applicant: Okada, Naoki, Asahi-ku Osaka-shi Osaka 535-0011 (JP)
(72) Inventor: Okada, Naoki, Asahi-ku Osaka-shi Osaka 535-0011 (JP)
(74) Representative: Kramer Barske Schmidtchen Patentanwälte PartG mbB
(86) International application number: PCT/JP2020/047802
(87) International publication number: WO 2021/080024

(57) **Abstract**

The present invention provides a severity evaluation apparatus that enables optimum distribution of medical resources to provide optimized medical care. A model generation apparatus 10 has a teaching data acquisition unit 31 operable to acquire teaching data 90 labeled with disease information indicative of a level of disease of a body region for a CT image and a model generation unit 32 operable to generate a disease information model 50 configured to output disease information in response to an input of a CT image with machine learning that uses the teaching data 90. The severity evaluation apparatus 20 has a measurement data acquisition unit 41 operable to acquire CT images 60 of a patient, a disease information acquisition unit 42 operable to acquire disease information 70 from each of the CT images 60 with use of the disease information model 50, and a severity calculation unit 43 operable to calculate a severity 75 of the patient based on the disease information 70 acquired by the disease information acquisition unit 42.

## Description

### Technical Field

The present invention relates to a severity evaluation apparatus and a model generation apparatus, and more particularly to a severity evaluation apparatus that evaluates a severity of a patient who needs emergency medical care.

### Background Art

Nowadays, the Japanese population has been rapidly aging. An increased number of patients have caused the shortage of medical doctors. All the countries of the world also suffer from the shortage of medical doctors. Particularly, emergency and critical care departments have been an infrastructure that is essential to general people because they shoulder at least six million cases of ambulance transport and off-hour medical service per year. Medical doctors tend to avoid emergency and critical medical care because extremely prompt and adequate treatment is required for the emergency and critical medical care. Accordingly, the shortage of medical doctors has been serious particularly in the field of emergency and critical care, which thus chronically lacks time and manpower.

The highest priority in initial treatment of such emergency and critical medical care is to accurately evaluate the severity of a patient. This is because it is to distinguish between a patient with mild disease and a patient with severe disease that enables optimum distribution of medical resources and can save many lives even with restrictions on time and manpower as described above. In actual conditions of emergency and critical care, however, patients are often transported one after another. Thus, it is difficult for emergency physicians to evaluate the severity of a large number of patients promptly and adequately with limited medical resources. Accordingly, there has been demanded immediate establishment of a system that can provide optimum emergency and critical medical care depending on the severity of each of patients.

### Summary of the Invention

### Problem(s) to be Solved by the Invention

The present invention has been made in view of the above drawbacks in the prior art. It is, therefore, an object of the present invention to provide a model generation apparatus and a severity evaluation apparatus that enables optimum distribution of medical resources to provide optimized medical care.

### Means for Solving Problem(s)

According to a first aspect of the present invention, there is provided a model generation apparatus that enables optimum distribution of medical resources to provide optimized medical care. The model generation apparatus has a teaching data acquisition unit operable to acquire, for measurement data obtained by measurement of a physical condition of a human, teaching data labeled with disease information indicative of information on a disease of a body region included in the measurement data and a disease information model generation unit operable to generate a disease information model with machine learning that uses the teaching data acquired by the teaching data acquisition unit, the disease information model being configured to output, in response to an input of measurement data obtained by measurement of a physical condition of a human, disease information of a body region included in the measurement data.

According to a second aspect of the present invention, there is provided a model generation apparatus that enables optimum distribution of medical resources to provide optimized medical care. The model generation apparatus has a teaching data acquisition unit operable to acquire, for measurement data obtained by measurement of a physical condition of a human, teaching data labeled with a classification of a body region included in the measurement data and a body region classification model generation unit operable to generate a body region classification model with machine learning that uses the teaching data acquired by the teaching data acquisition unit, the body region classification model being configured to output, in response to an input of measurement data obtained by measurement of a physical condition of a human, a classification of a body region included in the measurement data.

According to a third aspect of the present invention, there is provided a model generation apparatus that enables optimum distribution of medical resources to provide optimized medical care. The model generation apparatus has a teaching data acquisition unit operable to acquire, for measurement data obtained by measurement of a physical condition of a human, teaching data labeled with a classification of a body region included in the measurement data and disease information indicative of information on a disease of the body region and a disease information model generation unit operable to generate a disease information model with machine learning that uses the teaching data acquired by the teaching data acquisition unit, the disease information model being configured to output, in response to an input of measurement data obtained by measurement of a physical condition of a human and a classification of a body region included in the measurement data, disease information of the body region included in the measurement data.

According to a fourth aspect of the present invention, there is provided a severity evaluation apparatus that enables optimum distribution of medical resources to provide optimized medical care. The severity evaluation apparatus has a measurement data acquisition unit operable to acquire a plurality of sets of measurement data obtained by measurement of a physical condition of a patient, a disease information acquisition unit operable to acquire disease information of a body region included in each set of measurement data from the plurality of sets of measurement data acquired by the measurement data acquisition unit with use of a disease information model, the disease information model being configured to output, in response to an input of measurement data obtained by measurement of a physical condition of a human, disease information indicative of information on a disease of a body region included in the measurement data, and a severity calculation unit operable to calculate a severity of the patient based on the disease information acquired by the disease information acquisition unit.

According to a fifth aspect of the present invention, there is provided a severity evaluation apparatus that enables optimum distribution of medical resources to provide optimized medical care. The severity evaluation apparatus has a measurement data acquisition unit operable to acquire a plurality of sets of measurement data obtained by measurement of a physical condition of a patient, a body region classification acquisition unit operable to acquire a classification of a body region included in each set of measurement data from the plurality of sets of measurement data acquired by the measurement data acquisition unit with use of a body region classification model, the body region classification model being configured to output, in response to an input of measurement data obtained by measurement of a physical condition of a human, a classification of a body region included in the measurement data, a disease information acquisition unit operable to acquire disease information of a body region included in each set of measurement data from the plurality of sets of measurement data acquired by the measurement data acquisition unit and the classification of the body region acquired by the body region classification acquisition unit with use of a disease information model, the disease information model being configured to output, in response to an input of measurement data obtained by measurement of a physical condition of a human and a classification of a body region included in the measurement data, disease information of the body region included in the measurement data, and a severity calculation unit operable to calculate a severity of the patient based on the disease information acquired by the disease information acquisition unit.

### Brief Description of Drawings

Fig. 1 is a block diagram schematically showing an emergency and critical care support system including a severity evaluation apparatus according to a first embodiment of the present invention.
Fig. 2 is a block diagram showing an example of a hardware configuration of a model generation apparatus illustrated in Fig. 1.
Fig. 3 is a block diagram showing an example of a hardware configuration of a severity evaluation apparatus illustrated in Fig. 1.
Fig. 4 is a block diagram schematically showing an emergency and critical care support system including a severity evaluation apparatus according to a second embodiment of the present invention.

### Mode(s) for Carrying Out the Invention

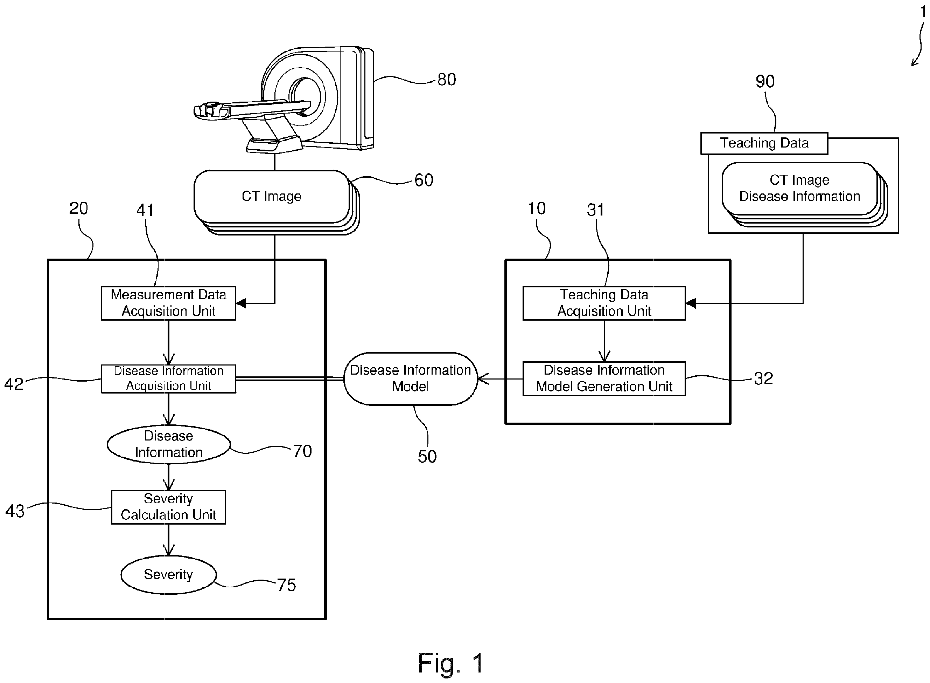

Embodiments of an emergency and critical care support system including a model generation apparatus and a severity evaluation apparatus according to the present invention will be described in detail below with reference to Figs. 1 to 4. In Figs. 1 to 4, the same or corresponding components are denoted by the same or corresponding reference numerals and will not be described below repetitively. Furthermore, in Figs. 1 to 4, the scales or dimensions of components may be exaggerated, or some components may be omitted. Unless mentioned otherwise, in the following description, terms such as "first," "second," etc. are only used to distinguish one component from another and are not used to indicate a specific order or a specific sequence.

Fig. 1 is a block diagram schematically showing an emergency and critical care support system 1 according to a first embodiment of the present invention. As shown in Fig. 1, the emergency and critical care support system 1 of the present embodiment includes a model generation apparatus 10 operable to generate a disease information model 50 with machine learning and a severity evaluation apparatus 20 operable to evaluate the severity of a patient from measurement data obtained by measurement of a physical condition of the patient, with use of the disease information model 50 generated by the model generation apparatus 10.

The present embodiment describes an example in which computerized tomography images (CT images) for internal parts of a patient body are used as measurement data obtained by measurement of a physical condition of a human. Nevertheless, usable measurement data are not limited to CT images. For example, measurement data may include magnetic resonance imaging (MRI) pictures, physiological function test data such as three-dimensional measurement data, collected blood data, electrocardiograms, and respiratory function data, X-ray examination data, ultrasonography data, data indicating sign of life, and other examination data. Alternatively, those data may be combined with each other.

The emergency and critical care support system 1 of the present embodiment includes a measurement device 80 operable to measure a physical condition of a patient. In the present embodiment, CT images are used as measurement data as described above. Therefore, a CT scanner for taking cross-sectional images of internal parts of a patient body using an X-ray is used as the measurement device 80. In a case where MRI pictures are used as the measurement data, an MRI scanner for imaging information on internal parts of a patient body using a magnetic resonance phenomenon is used as the measurement device 80. Furthermore, if other examination data are used as measurement data, an examination device for obtaining data describing physical information of a patient during each examination is used as the measurement device 80.

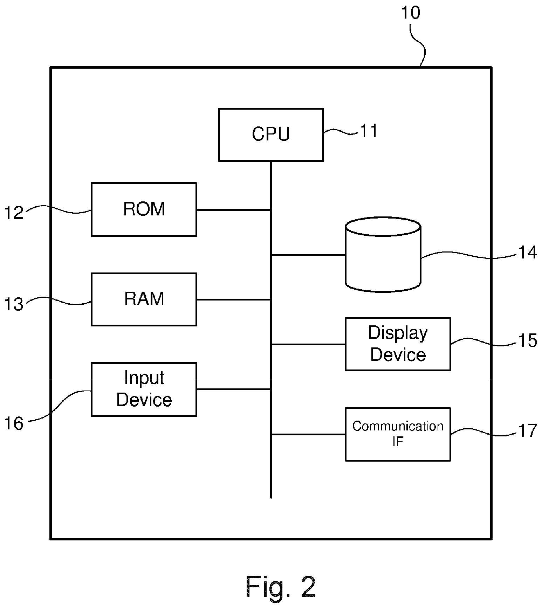

Fig. 2 is a block diagram showing an example of a hardware configuration of the model generation apparatus 10. The model generation apparatus 10 may be formed by, for example, a server computer, a general computer, a dedicated computer, a portable terminal, or the like. Those devices may be combined with each other to form the model generation apparatus 10. The model generation apparatus 10 may share hardware with another device.

As shown in Fig. 2, the model generation apparatus 10 has a central processing unit (CPU) 11, a read-only memory (ROM) 12, a random-access memory (RAM) 13, a storage device 14 such as a hard disk or a solid-state disk, a display device 15 such as a display unit, an input device 16 such as a keyboard or a mouse, and a communication interface 17 that enables communication with other devices. The CPU 11 reads and executes programs stored in the ROM 12, the RAM 13, or the storage device 14 to implement various functional units as described below. The communication interface 17 controls wired or wireless communication with other devices. The display device 15 and the input device 16 may be formed by a device having both of a display function and an input function, such as a touch panel.

As shown in Fig. 1, the model generation apparatus 10 includes, as functional units implemented by the aforementioned programs, a teaching data acquisition unit 31 operable to acquire teaching data 90 as described below and a disease information model generation unit 32 operable to generate a disease information model as described below with machine learning that uses the teaching data 90 acquired by the teaching data acquisition unit 31.

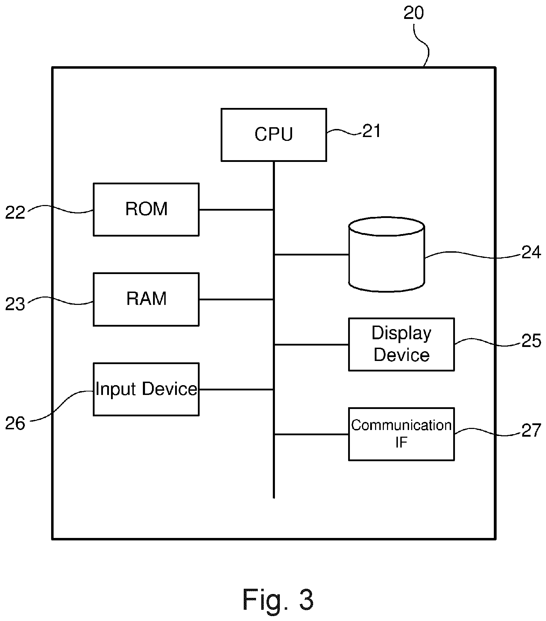

Fig. 3 is a block diagram showing an example of a hardware configuration of the severity evaluation apparatus 20. The severity evaluation apparatus 20 may be formed by, for example, a server computer, a general computer, a dedicated computer, a portable terminal, or the like. Those devices may be combined with each other to form the severity evaluation apparatus 20. The severity evaluation apparatus 20 may share hardware with another device.

As shown in Fig. 3, the severity evaluation apparatus 20 has a central processing unit (CPU) 21, a read-only memory (ROM) 22, a random-access memory (RAM) 23, a storage device 24 such as a hard disk or a solid-state disk, a display device 25 such as a display unit, an input device 26 such as a keyboard or a mouse, and a communication interface 27 that enables communication with other devices. The CPU 21 reads and executes programs stored in the ROM 22, the RAM 23, or the storage device 24 to implement various functional units as described below. The communication interface 27 controls wired or wireless communication with other devices. The display device 25 and the input device 26 may be formed by a device having both of a display function and an input function, such as a touch panel.

As shown in Fig. 1, the severity evaluation apparatus 20 includes, as functional units implemented by the aforementioned programs, a measurement data acquisition unit 41, a disease information acquisition unit 42, and a severity calculation unit 43. The measurement data acquisition unit 41 of the severity evaluation apparatus 20 is configured to be connectable to the measurement device 80 via the aforementioned communication interface 27. The measurement data acquisition unit 41 of the severity evaluation apparatus 20 and the measurement device 80 may be connected to each other via a network such as a local area network (LAN), a wide area network (WAN), a near-field communication network, an intranet, or the Internet.

Before operation of the emergency and critical care support system 1, for a number of past CT images, information on disease of a body region (e.g., a level of disease) that can be found in each of the CT images is labeled as disease information. Teaching data 90 labeled with disease information are generated for a number of CT images. The teaching data 90 are stored in a device or a recording medium connected to the model generation apparatus 10 via a network, or in the storage device 14 within the model generation apparatus 10.

Examples of the disease information used for labeling the teaching data 90 may include a level of disease. In this case, a level of disease may be determined for surgical evaluation, for example, depending on the degree of injury to a body region. A level of disease may be determined for medical evaluation, for example, depending on the degree of a lesion or a size of an inflammation in a body region. For example, three labels of "mild disease," "moderate disease," and "severe disease" may be used for the disease information indicative of such a level of disease. Furthermore, the disease information may employ a condition of a patient when the patient came to a hospital, disease course during hospitalization (e.g., patient's prognosis classified by disease course during hospitalization (remission, brain death, death, etc.) or the like), a treatment provided (whether to have undergone an operation, whether to have dosed a drug, the amount of medicine, etc.), or the like.

The teaching data acquisition unit 31 of the model generation apparatus 10 is operable to acquire the teaching data 90 generated as described above from the external device via the communication interface 17 or read the teaching data 90 from the recording medium or the storage device 14. The teaching data 90 acquired by the teaching data acquisition unit 31 are used in machine learning on the disease information model generation unit 32. The disease information model generation unit 32 is operable to generate, through machine learning using the teaching data 90, a disease information model 50 that can output disease information of a body region that is found in a CT image when the CT image is inputted. The machine learning method performed in the disease information model generation unit 32 is not limited to a specific one and may be, for example, machine learning using a neural network. The disease information model 50 generated in the disease information model generation unit 32 is stored in a device or a storage medium connected to the severity evaluation apparatus 20 via a network or in the storage device 24 within the severity evaluation apparatus 20.

With the severity evaluation apparatus 20, use of such a disease information model 50 allows, for example, the severity of a patient transported by an ambulance to be evaluated automatically in a short period of time. More specifically, when a patient is transported by an ambulance, CT images 60 are taken for the whole body of the patient by the measurement device 80. The measurement data acquisition unit 41 of the severity evaluation apparatus 20 acquires those CT images 60 from the measurement device 80 and transmits the CT images 60 to the disease information acquisition unit 42.

The disease information acquisition unit 42 is operable to acquire the disease information model 50 generated by the model generation apparatus 10 from the external device on the network via the communication interface 17 or read the disease information model 50 from the storage medium or the storage device 24. Then the disease information acquisition unit 42 inputs the CT images 60 acquired from the measurement device 80 to the disease information model 50 and retrieves the disease information 70 on each of the CT images 60 as outputs.

The severity calculation unit 43 of the severity evaluation apparatus 20 is operable to calculate a comprehensive severity 75 of the patient based on the disease information 70 on each of the CT images 60 that has been acquired by the disease information acquisition unit 42. The calculation method for the comprehensive severity 75 may be obtained empirically by emergency physicians or may be based on specific guidelines or theses.

The severity calculation unit 43 may also calculate a severity 75 in consideration of other additional information in addition to the disease information 70 acquired by the disease information acquisition unit 42. Examples of such additional information include body findings, other measurement data (e.g., a blood pressure, a body temperature, a pulse, a percutaneous arterial oxygen saturation (SpO₂), three-dimensional measurement data, collected blood data, physiological function data, X-ray examination data, ultrasonography data, data indicating sign of life, etc.) and data relating to a cause of disease of a patient (information from ambulance crews (e.g., a cause of disease of a patient (a traffic accident, a fall, a fire, etc.), a situation of an accident, eyewitness information, body findings, and measurement data from a biometric monitor), information from a medical doctor who was in charge in the past, a medical chart, a past examination result, etc.).

The severity 75 of the patient thus calculated is displayed on the display device 15 such as a display unit. Thus, an emergency physician can instantly know the comprehensive severity of the patient. Furthermore, the number of days required for hospitalization or a medial treatment cost may be estimated from the calculated severity 75 and additionally displayed on the display device 15 such as a display unit.

In this manner, according to the severity evaluation apparatus 20 of the present embodiment, a comprehensive severity 75 of a patient can automatically be evaluated from CT images 60 of the whole body of the patient in a short period of time. Therefore, an emergency physician can instantly know the comprehensive severity of the patient. Thus, the emergency physician can readily determine the priority of treatments to respective patients. Accordingly, medical resources can optimally be distributed depending on the severity of the respective patients. Furthermore, the emergency physician does not need to spend time on evaluation of the severity of patients and can spend more time on treatment of patients. Thus, more lives can be saved.

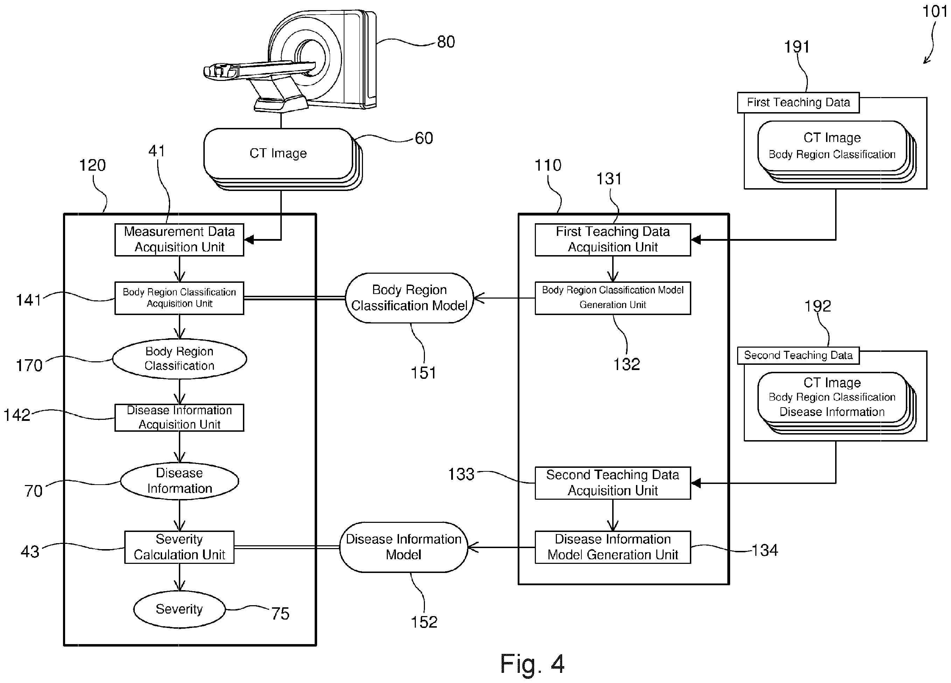

Fig. 4 is a block diagram schematically showing an emergency and critical care support system 101 according to a second embodiment of the present invention. As shown in Fig. 4, the emergency and critical care support system 101 of the present embodiment includes a model generation apparatus 110 operable to generate a body region classification model 151 and a disease information model 152 with machine learning and a severity evaluation apparatus 120 operable to evaluate the severity of a patient from measurement data (e.g., CT images) obtained by measurement of a physical condition of the patient, with use of the body region classification model 151 and the disease information model 152 generated by the model generation apparatus 110.

The model generation apparatus 110 of the present embodiment has a hardware configuration similar to that of the model generation apparatus 10 as shown in Fig. 2. As illustrated in Fig. 4, the model generation apparatus 110 includes a first teaching data acquisition unit 131 operable to acquire first teaching data 191 as described below, a body region classification model generation unit 132 operable to generate a body region classification model 151 as described below with machine learning that uses the first teaching data 191 acquired by the first teaching data acquisition unit 131, a second teaching data acquisition unit 133 operable to acquire second teaching data 192 as described below, and a disease information model generation unit 134 operable to generate a disease information model 152 as described below with machine learning that uses the second teaching data 192 acquired by the second teaching data acquisition unit 133.

The severity evaluation apparatus 120 of the present embodiment has a hardware configuration similar to that of the severity evaluation apparatus 20 as shown in Fig. 3. As illustrated in Fig. 4, the severity evaluation apparatus 120 includes a body region classification acquisition unit 141 and a disease information acquisition unit 142 in addition to the measurement data acquisition unit 41 and the severity calculation unit 43 of the first embodiment.

The first teaching data 191 used in the present embodiment are labeled with anatomical classifications for a body region found in each of a number of past CT images (e.g., "liver," "pancreas," "spleen," etc.). The second teaching data 192 are labeled with anatomical classifications for a body region found in each of a number of past CT images along with information on disease of the body region (e.g., a level of disease) as disease information.

The first teaching data 191 and the second teaching data 192 are stored in a device or a recording medium connected to the model generation apparatus 110 via a network, or in the storage device 14 (see Fig. 2) within the model generation apparatus 110. Classifications for body regions that are used as labels in the first teaching data 191 and the second teaching data 192 may be anatomical classifications as described above. Alternatively, other classifications may be used for such classifications. For example, a body of a patient may be classified into large classifications, such as "a head," "a chest," and "an abdomen." Those large classifications may further be classified into small classifications, such as "a bone" and "a blood vessel." The first teaching data 191 and the second teaching data 192 may be labeled with multilayered classifications.

The first teaching data acquisition unit 131 of the model generation apparatus 110 is operable to acquire the first teaching data 191 from the external device via the communication interface 17 or read the first teaching data 191 from the recording medium or the storage device 14. The first teaching data 191 acquired by the first teaching data acquisition unit 131 are used in machine learning on the body region classification model generation unit 132. The body region classification model generation unit 132 is operable to generate, through machine learning using the first teaching data 191, a body region classification model 151 that can output a classification of a body region found in a CT image when the CT image is inputted. The machine learning method performed in the body region classification model generation unit 132 is not limited to a specific one and may be, for example, machine learning using a neural network. The body region classification model 151 generated in the body region classification model generation unit 132 is stored in a device or a storage medium connected to the severity evaluation apparatus 120 via a network or in the storage device 24 within the severity evaluation apparatus 120.

The second teaching data acquisition unit 133 of the model generation apparatus 110 is operable to acquire the second teaching data 192 from the external device via the communication interface 17 or read the second teaching data 192 from the recording medium or the storage device 14. The second teaching data 192 acquired by the second teaching data acquisition unit 133 are used in machine learning on the disease information model generation unit 134. The disease information model generation unit 134 is operable to generate, through machine learning using the second teaching data 192, a disease information model 152 that can output disease information when a CT image is inputted along with a classification of a body region found in the CT image. The machine learning method performed in the disease information model generation unit 134 is not limited to a specific one and may be, for example, machine learning using a neural network. The disease information model 152 generated in the disease information model generation unit 134 is stored in a device or a storage medium connected to the severity evaluation apparatus 120 via a network or in the storage device 24 within the severity evaluation apparatus 120.

With the severity evaluation apparatus 120, CT images 60 taken for the whole body of a patient by the measurement device 80 are acquired by the measurement data acquisition unit 41 and transmitted to the body region classification acquisition unit 141. The body region classification acquisition unit 141 inputs the CT images 60 acquired from the measurement device 80 to the aforementioned body region classification model 151 and retrieves a classification (e.g., anatomical classification) 170 of the body region for each of the CT images 60 as outputs. Then the disease information acquisition unit 142 inputs the CT images 60 acquired from the measurement device 80 along with the classification 170 retrieved by the body region classification acquisition unit 141 to the aforementioned disease information model 152 and retrieves disease information 70 for each of the CT images 60 as outputs. The severity calculation unit 43 of the severity evaluation apparatus 120 calculates a comprehensive severity 75 of the patient based on the disease information 70 for each of the CT images 60 that has been acquired by the disease information acquisition unit 142.

The severity 75 of the patient thus calculated is displayed on the display device 15 such as a display unit. Thus, an emergency physician can instantly know the comprehensive severity of the patient. In this case, the severity calculation unit 43 of the severity evaluation apparatus 120 may be configured to calculate the severity 75 of the patient for each of the classifications 170 acquired by the body region classification acquisition unit 141. Such a configuration allows an emergency physician to instantly know the severity of a patient transported by an ambulance for each of the classifications of the body regions (in other words, each of organs). Therefore, the emergency physician can readily determine the priority of organs to be treated among the organs of the patient. Thus, a possibility of saving a life of the patient can be increased.

Although some preferred embodiments of the present invention have been described, the present invention is not limited to the aforementioned embodiments. It should be understood that various different forms may be applied to the present invention within the technical idea thereof.

As described above, according to a first aspect of the present invention, there is provided a model generation apparatus that enables optimum distribution of medical resources to provide optimized medical care. The model generation apparatus has a teaching data acquisition unit operable to acquire, for measurement data obtained by measurement of a physical condition of a human, teaching data labeled with disease information indicative of information on a disease of a body region included in the measurement data and a disease information model generation unit operable to generate a disease information model with machine learning that uses the teaching data acquired by the teaching data acquisition unit, the disease information model being configured to output, in response to an input of measurement data obtained by measurement of a physical condition of a human, disease information of a body region included in the measurement data.

According to a second aspect of the present invention, there is provided a model generation apparatus that enables optimum distribution of medical resources to provide optimized medical care. The model generation apparatus has a teaching data acquisition unit operable to acquire, for measurement data obtained by measurement of a physical condition of a human, teaching data labeled with a classification of a body region included in the measurement data and a body region classification model generation unit operable to generate a body region classification model with machine learning that uses the teaching data acquired by the teaching data acquisition unit, the body region classification model being configured to output, in response to an input of measurement data obtained by measurement of a physical condition of a human, a classification of a body region included in the measurement data.

According to a third aspect of the present invention, there is provided a model generation apparatus that enables optimum distribution of medical resources to provide optimized medical care. The model generation apparatus has a teaching data acquisition unit operable to acquire, for measurement data obtained by measurement of a physical condition of a human, teaching data labeled with a classification of a body region included in the measurement data and disease information indicative of information on a disease of the body region and a disease information model generation unit operable to generate a disease information model with machine learning that uses the teaching data acquired by the teaching data acquisition unit, the disease information model being configured to output, in response to an input of measurement data obtained by measurement of a physical condition of a human and a classification of a body region included in the measurement data, disease information of the body region included in the measurement data.

According to a fourth aspect of the present invention, there is provided a severity evaluation apparatus that enables optimum distribution of medical resources to provide optimized medical care. The severity evaluation apparatus has a measurement data acquisition unit operable to acquire a plurality of sets of measurement data obtained by measurement of a physical condition of a patient, a disease information acquisition unit operable to acquire disease information of a body region included in each set of measurement data from the plurality of sets of measurement data acquired by the measurement data acquisition unit with use of a disease information model, the disease information model being configured to output, in response to an input of measurement data obtained by measurement of a physical condition of a human, disease information indicative of information on a disease of a body region included in the measurement data, and a severity calculation unit operable to calculate a severity of the patient based on the disease information acquired by the disease information acquisition unit.

According to a fifth aspect of the present invention, there is provided a severity evaluation apparatus that enables optimum distribution of medical resources to provide optimized medical care. The severity evaluation apparatus has a measurement data acquisition unit operable to acquire a plurality of sets of measurement data obtained by measurement of a physical condition of a patient, a body region classification acquisition unit operable to acquire a classification of a body region included in each set of measurement data from the plurality of sets of measurement data acquired by the measurement data acquisition unit with use of a body region classification model, the body region classification model being configured to output, in response to an input of measurement data obtained by measurement of a physical condition of a human, a classification of a body region included in the measurement data, a disease information acquisition unit operable to acquire disease information of a body region included in each set of measurement data from the plurality of sets of measurement data acquired by the measurement data acquisition unit and the classification of the body region acquired by the body region classification acquisition unit with use of a disease information model, the disease information model being configured to output, in response to an input of measurement data obtained by measurement of a physical condition of a human and a classification of a body region included in the measurement data, disease information of the body region included in the measurement data, and a severity calculation unit operable to calculate a severity of the patient based on the disease information acquired by the disease information acquisition unit. The severity calculation unit may be configured to calculate the severity of the patient for each classification of the body region.

According to a configuration in one aspect of the present invention, with use of a disease information model that can output disease information of a body region included in measurement data, a severity of a patient can automatically be evaluated in a short period of time from a plurality of sets of measurement data obtained by measurement of a physical condition of the patient, so that a medical doctor can instantly know the severity of the patient. Accordingly, medical resources can optimally be distributed depending on the severity of respective patients. Thus, optimized medical care can be provided.

The severity calculation unit may be configured to calculate the severity of the patient in further consideration of at least one of a body finding, other measurement data (e.g., a blood pressure, a body temperature, a pulse, a percutaneous arterial oxygen saturation (SpO₂), three-dimensional measurement data, collected blood data, physiological function data, X-ray examination data, ultrasonography data, data indicating sign of life, etc.), and data relating to a cause of disease of a patient (information from ambulance crews (e.g., a cause of disease of a patient (a traffic accident, a fall, a fire, etc.), a situation of an accident, eyewitness information, body findings, and measurement data from a biometric monitor), information from a medical doctor who was in charge in the past, a medical chart, a past examination result, etc.). The measurement data may include at least one of a CT image, an MRI picture, three-dimensional measurement data, collected blood data, physiological function test data such as electrocardiogram and respiratory function data, X-ray examination data, ultrasonography data, and data indicating sign of life.

According to one aspect of the present invention, with use of a disease information model that can output disease information of a body region included in measurement data, a severity of a patient can automatically be evaluated in a short period of time from a plurality of sets of measurement data obtained by measurement of a physical condition of the patient, so that a medical doctor can instantly know the severity of the patient. Accordingly, medical resources can optimally be distributed depending on the severity of respective patients. Thus, optimized medical care can be provided.

This application claims the benefit of priority from Japanese patent application No. 2019-194633, filed on October 25, 2019, the disclosure of which is incorporated herein in its entirety by reference.

### Industrial Applicability

The present invention is suitably used for a severity evaluation apparatus that evaluates a severity of a patient who needs emergency and critical care.

### Description of Reference Numerals and Signs

- 1: Emergency and critical care support system
- 10: Model generation apparatus
- 20: Severity evaluation apparatus
- 31: Teaching data acquisition unit
- 32: Disease information model generation unit
- 41: Measurement data acquisition unit
- 42: Disease information acquisition unit
- 43: Severity calculation unit
- 50: Disease information model
- 60: CT image
- 70: Disease information
- 75: Severity
- 80: Measurement device
- 90: Teaching data
- 101: Emergency and critical care support system
- 110: Model generation apparatus
- 120: Severity evaluation apparatus
- 131: First teaching data acquisition unit
- 132: Body region classification model generation unit
- 133: Second teaching data acquisition unit
- 134: Disease information model generation unit
- 141: Body region classification acquisition unit
- 142: Disease information acquisition unit
- 151: Body region classification model
- 152: Disease information model
- 191: First teaching data
- 192: Second teaching data

## Claims

1. A model generation apparatus comprising:
a teaching data acquisition unit operable to acquire, for measurement data obtained by measurement of a physical condition of a human, teaching data labeled with disease information indicative of information on a disease of a body region included in the measurement data; and
a disease information model generation unit operable to generate a disease information model with machine learning that uses the teaching data acquired by the teaching data acquisition unit, the disease information model being configured to output, in response to an input of measurement data obtained by measurement of a physical condition of a human, disease information of a body region included in the measurement data.

2. A model generation apparatus comprising:
a teaching data acquisition unit operable to acquire, for measurement data obtained by measurement of a physical condition of a human, teaching data labeled with a classification of a body region included in the measurement data; and
a body region classification model generation unit operable to generate a body region classification model with machine learning that uses the teaching data acquired by the teaching data acquisition unit, the body region classification model being configured to output, in response to an input of measurement data obtained by measurement of a physical condition of a human, a classification of a body region included in the measurement data.

3. A model generation apparatus comprising:
a teaching data acquisition unit operable to acquire, for measurement data obtained by measurement of a physical condition of a human, teaching data labeled with a classification of a body region included in the measurement data and disease information indicative of information on a disease of the body region; and
a disease information model generation unit operable to generate a disease information model with machine learning that uses the teaching data acquired by the teaching data acquisition unit, the disease information model being configured to output, in response to an input of measurement data obtained by measurement of a physical condition of a human and a classification of a body region included in the measurement data, disease information of the body region included in the measurement data.

4. The model generation apparatus as recited in any one of claims 1 to 3, wherein the measurement data include at least one of a CT image, an MRI picture, three-dimensional measurement data, collected blood data, physiological function test data, X-ray examination data, ultrasonography data, and data indicating sign of life.

5. A severity evaluation apparatus comprising:
a measurement data acquisition unit operable to acquire a plurality of sets of measurement data obtained by measurement of a physical condition of a patient;
a disease information acquisition unit operable to acquire disease information of a body region included in each set of measurement data from the plurality of sets of measurement data acquired by the measurement data acquisition unit with use of a disease information model, the disease information model being configured to output, in response to an input of measurement data obtained by measurement of a physical condition of a human, disease information indicative of information on a disease of a body region included in the measurement data; and
a severity calculation unit operable to calculate a severity of the patient based on the disease information acquired by the disease information acquisition unit.

6. A severity evaluation apparatus comprising:
a measurement data acquisition unit operable to acquire a plurality of sets of measurement data obtained by measurement of a physical condition of a patient;
a body region classification acquisition unit operable to acquire a classification of a body region included in each set of measurement data from the plurality of sets of measurement data acquired by the measurement data acquisition unit with use of a body region classification model, the body region classification model being configured to output, in response to an input of measurement data obtained by measurement of a physical condition of a human, a classification of a body region included in the measurement data;
a disease information acquisition unit operable to acquire disease information of a body region included in each set of measurement data from the plurality of sets of measurement data acquired by the measurement data acquisition unit and the classification of the body region acquired by the body region classification acquisition unit with use of a disease information model, the disease information model being configured to output, in response to an input of measurement data obtained by measurement of a physical condition of a human and a classification of a body region included in the measurement data, disease information of the body region included in the measurement data; and
a severity calculation unit operable to calculate a severity of the patient based on the disease information acquired by the disease information acquisition unit.

7. The severity evaluation apparatus as recited in claim 6, wherein the severity calculation unit is configured to calculate the severity of the patient for each classification of the body region.

8. The severity evaluation apparatus as recited in any one of claims 5 to 7, wherein the severity calculation unit is configured to calculate the severity of the patient in further consideration of at least one of a body finding, other measurement data, and data relating to a cause of disease of a patient.

9. The severity evaluation apparatus as recited in any one of claims 5 to 8, wherein the measurement data include at least one of a CT image, an MRI picture, three-dimensional measurement data, collected blood data, physiological function test data, X-ray examination data, ultrasonography data, and data indicating sign of life.
